# EUROPÄISCHE PATENTSCHRIFT

(11) **EP 3 620 446 B1**
(45) Veröffentlichungstag und Bekanntmachung des Hinweises auf die Patenterteilung: **28.06.2023**
(21) Anmeldenummer: 19195286.0
(22) Anmeldetag: 04.09.2019
(51) Int. Cl.: C07C 41/50, C07C 43/30, B01J 23/72, B01J 23/42, B01J 23/52, B01J 27/051, B01J 27/14, B01J 29/14, B01J 29/76

(54) **VERFAHREN ZUR HERSTELLUNG VON DIMETHOXYMETHAN**
METHOD FOR PRODUCING DIMETHOXYMETHANE
PROCÉDÉ DE FABRICATION DE DIMÉTHOXYMÈTHANE

(30) Priorität: 05.09.2018 DE 102018121607
(43) Veröffentlichungstag der Anmeldung: 11.03.2020
(73) Patentinhaber: Rheinisch-Westfälische Technische Hochschule (RWTH) Aachen, 52056 Aachen (DE)
(72) Erfinder: Palkovits, Regina, 52074 Aachen (DE); Sun, Ruiyan, 52074 Aachen (DE); Gierlich, Christian, 52074 Aachen (DE)
(74) Vertreter: Michalski Hüttermann & Partner Patentanwälte mbB

(56) Entgegenhaltungen:
- US-A- 2 663 742
- YUTAKA MORIKAWA ET AL: "DIFFERENCE IN THE CATALYTIC ACTIVITIES FOR THE CONVERSION OF METHANOL BETWEEN TRIVALENT AND TETRAVALENT Ti ION EXCHANGED FORMS OF LAYERED SILICATE", CHEMISTRY LETTERS, Bd. 12, Nr. 12, 5. Dezember 1983 (1983-12-05), Seiten 1849-1852, XP55655525, JAPAN ISSN: 0366-7022, DOI: 10.1246/cl.1983.1849
- LIUBI WU ET AL: "Direct Synthesis of Hydrogen and Dimethoxylmethane from Methanol on Copper/Silica Catalysts with Optimal Cu + /Cu 0 Sites", CHEMCATCHEM, Bd. 10, Nr. 5, 7. März 2018 (2018-03-07), Seiten 1140-1147, XP55655380, DE ISSN: 1867-3880, DOI: 10.1002/cctc.201701416

## Beschreibung

Die Erfindung betrifft ein Verfahren zur Herstellung von Dimethoxymethan aus Methanol.

Dimethoxymethan, üblicherweise als Methylal bezeichnet, ist mit n = 1 das kleinste Oligomer der Oxymethylenether der chemischen Struktur H₃C-O-(CH₂O)ₙ-CH₃. Dimethoxymethan ist eine klare, farblose und entzündliche Flüssigkeit mit niedrigem Siedepunkt, niedriger Viskosität und ist mit den meisten organischen Lösungsmitteln mischbar. Dimethoxymethan wird vor allem als Lösemittel und in der Produktion von Parfüm, Harzen, Farblösern und Schutzanstrichen verwendet. Dimethoxymethan lässt sich ferner zu weiteren Oxymethylenethern umsetzen. Kurzkettige Oxymethylenether lassen sich in beliebigen Mengen mit Dieselkraftstoff mischen und insbesondere Oxymethylenether mit n = 3 bis 5 werden derzeit als Zusatzstoffe bzw. langfristig als Alternative zu Dieselkraftstoff diskutiert.

Die oxidative Herstellung von Dimethoxymethan aus Methanol ist seit längerer Zeit bekannt, beispielsweise aus der US 2,663,742. Auch die CN 103848727 A und CN 101428211 A beschreiben Verfahren und Katalysatoren zur oxidativen Herstellung von Dimethoxymethan aus Methanol. Nicht-oxidative Verfahren, bei welchen Methanol als Edukt verwendet wird, werden üblicherweise verwendet, um Methylformiat oder Formaldehyd als Produkt zu erhalten. So beschreibt die EP 0 427 062 A2 ein Verfahren zur Herstellung von Methylformiat durch Umsetzung von Methanol an kupferhaltigen Katalysatoren bei erhöhter Temperatur in der Gasphase. Die DE 19814281 A1 beschreibt ein Verfahren zur nicht-oxidativen Herstellung von Formaldehyd aus Methanol. Ebenso beschreiben die US 6232507 B1 und die WO 00/12471 A1 Verfahren zur Herstellung von Formaldehyd aus Methanol durch nicht-oxidative Dehydrierung in einem Reaktor in Gegenwart eines Katalysators.

Wu et al. beschreiben in ChemCatChem, 2018, 10, 1140-1147 die nicht-oxidative Herstellung von Dimethoxymethan und Wasserstoff aus Methanol an Kupfer/Siliziumoxid-Katalysatoren. Hierbei lag das Methanol in der flüssigen Phase vor. Flüssigphasen-Umsetzungen weisen den Nachteil auf, dass Reaktionsprozesse insgesamt langsam ablaufen. Zudem sind derartige Verfahren energetisch ungünstig, da sie unter hohem Druck und bei hohen Temperaturen eingesetzt werden, wodurch die Verfahren generell mit hohen Kosten verbunden sind. So verwenden Wu et al. überkritisches Methanol bei 2 MPa. Insgesamt sind diese Reaktionsbedingungen für eine Aufskalierung und Produktion in industriellem Umfang ungeeignet. Bisher ist kein nicht-oxidatives Syntheseverfahren bekannt, das effizient eine industrielle Produktion von Dimethoxymethan aus Methanol erlaubt. Es besteht ein Bedarf an alternativen nicht-oxidativen Herstellungsverfahren für Dimethoxymethan aus Methanol.

Morikawa et al., Chem. Lett., Band 12, Nr. 12, Seiten 1849 - 1852, "Difference in the catalytic activities for the conversion of methanol between trivalent and tetravalent Ti ion exchanged forms of layered silicate" beschreiben Unterschiede in den katalytischen Aktivitäten zwischen mit dreiwertigen und vierwertigen Ti-Ionen-ausgetauschten Formen von Schichtsilikaten für die Umwandlung von Methanol.

Der vorliegenden Erfindung lag die Aufgabe zu Grunde, ein Verfahren für Herstellung von Dimethoxymethan aus Methanol zur Verfügung zu stellen, dass mindestens einen der vorgenannten Nachteile des Standes der Technik überwindet.

Diese Aufgabe wird gelöst durch ein Verfahren zur nicht-oxidativen Herstellung von Dimethoxymethan aus Methanol in Gegenwart eines heterogenen Katalysators, wobei man gasförmiges Methanol mit dem Katalysator in Kontakt bringt. Hierbei ist der Katalysator ein bifunktioneller Katalysator umfassend:
a) Molybdänsulfid, oder
b)
   - ein Metall oder eine Metallverbindung ausgewählt aus der Gruppe umfassend Kupfer, Silber, Platin und/oder Molybdänsulfid, und
   - ein Alumosilikat, vorzugsweise ein Zeolith, oder ein Metalloxid ausgewählt aus der Gruppe umfassend Titanoxid, Zirkonoxid und/oder Aluminiumoxid.

Überraschend wurde gefunden, dass das erfindungsgemäße Verfahren die Umsetzung von Methanol zu Dimethoxymethan in der Gasphase erlaubt. Dies ist insbesondere überraschend, weil aufgrund thermodynamischer Berechnungen angenommen wurde, dass die Entstehung von Dimethoxymethan in der Gasphase nicht favorisiert sei. Vorteilhaft ist insbesondere, dass das Verfahren eine Umsetzung mit sehr kurzen Reaktionszeiten zur Verfügung stellt, das industriell umsetzbar ist. Die heterogenen Katalysatoren können nach der Reaktion durch einfache Verfahren wie Filtration abgetrennt und das Reaktionsgemisch kann aufgetrennt und weiterverarbeitet werden.

Insbesondere kann ein einschrittiges Verfahren zur Herstellung von Dimethoxymethan zur Verfügung gestellt werden. Ohne auf eine bestimmte Theorie festgelegt zu sein, wird davon ausgegangen, dass zunächst eine Dehydrierung des Methanols erfolgt und das entstehende Formaldehyd in der Umgebung von Methanol als Hemiacetal vorliegt. In einem nächsten Schritt wird das Hemiacetal mit Methanol zu Dimethoxymethan an einem sauren Katalysator kondensiert. Der Wasserstoff aus der Dehydrierung von Methanol ist wiederum für die Herstellung des Eduktes Methanol durch Hydrierung von Kohlendioxid nutzbar. Insbesondere im Vergleich zu der industriell genutzten oxidativen Herstellung, die zusätzliche Energie für die Elektrolyse von Wasser zu Wasserstoff beansprucht, erlaubt das erfindungsgemäße Verfahren eine Nutzung des entstehenden Wasserstoffs für die Herstellung des Eduktes. Die Rückführung des Wasserstoffs in die Produktionskette von Methanol und Dimethoxymethan erlaubt damit einen zirkulären Prozess, der insgesamt eine deutliche Einsparung von Energie und Kosten ermöglicht. Zudem weist das Verfahren durch die Abwesenheit von Sauerstoff und dem resultierenden Explosionsrisiko von Methanol an der Luft ein deutlich geringeres Gefahrenpotential auf, als die bislang industriell genutzte oxidative Herstellung von Dimethoxymethan.

Insbesondere unter Verwendung modifizierter Zeolithe konnten milde Reaktionsbedingungen von 150-200 °C bei guter Selektivität der Umsetzung für Dimethoxymethan erzielt werden. Hierbei können die Reaktionszeiten zur Optimierung der Raum-Zeit-Ausbeute unter einer Sekunde liegen. Diese Parameter erlauben eine Herstellung von Dimethoxymethan im industriellen Maßstab aus einem gut verfügbaren Edukt. Dies erlaubt darauf aufbauend eine weitere Umsetzung von Dimethoxymethan zu höheren Oxymethylenethern, welche ein großes Marktpotential insbesondere im Kraftstoff- und Kunststoffsektor besitzen.

In bevorzugten Ausführungsformen
sind das Alumosilikat und das Metalloxid optional mit Phospohor oder Schwefel dotiert.

Unter einem bifunktionellen Katalysator wird ein Katalysator verstanden, der zwei verschiedene aktive Zentrenarten mit unterschiedlicher Funktion umfasst, die einander zum katalytisch wirkenden System ergänzen. Ohne auf eine bestimmte Theorie festgelegt zu sein, wird davon ausgegangen, dass die Bifunktionalität des Katalysators wesentlich für die Umsetzung des Methanols zu Dimethoxymethan ist. Vorzugsweise umfasst der Katalysator ein metallisches und ein azides aktives Zentrum. Das metallische Zentrum kann durch Kupfer, Silber, Platin und/oder Molybdänsulfid ausgebildet sein. Insbesondere bevorzugt ist Kupfer. So zeigten bifunktionelle Katalysatoren umfassend Kupfer und Zeolith, Titanoxid oder Siliziumoxid, eine gute Selektivität und/oder Umsatz.

Das azide Zentrum kann durch Molybdänsulfid oder durch ein Alumosilikat (Zeolith) oder Metalloxid ausgewählt aus Titanoxid, Zirkonoxid und Aluminiumoxid ausgebildet sein. Insbesondere das Metalloxid wie Titanoxid und Aluminiumoxid kann mit ≥ 0,3 Gew.-% bis ≤ 1,5 Gew.-%, vorzugsweise ≥ 1 Gew.-% bis ≤ 1,5 Gew.-% Phospohor oder Schwefel, bezogen auf das Metalloxid, dotiert sein. Insbesondere Zeolithe können ohne Dotierung gute Ergebnisse erzielen. Bevorzugt sind Zeolithe und Metalloxide ausgewählt aus Titanoxid und/oder Aluminiumoxid. Besonders bevorzugt sind Zeolithe.

Unter dem Begriff "Zeolith" werden kristalline Alumosilikate, ausgebildet aus einer offenen dreidimensionalen Grundstruktur aus [SiO₄]⁴⁻- und [AlO₄]⁵⁻-Tetraedern verstanden. Diese Tetraeder sind über verbrückende Sauerstoffatome miteinander verbunden. Die negative Überschussladung im Anionengitter, hervorgerufen durch den Aluminiumgehalt, wird durch frei bewegliche, austauschbare Kationen wie Alkali- und Erdalkaliionen, wie Natrium, Kalium, Magnesium oder Calcium, ausgeglichen. Erfolgt der Ladungsausgleich ganz oder teilweise durch Protonen, werden die Zeolithe als "azide" Zeolithe bezeichnet. Die Säurestärke oder Azidität eines Zeolithen ist durch Ionenaustausch der Kationen einstellbar sowie weiter durch den Einbau von aziden Zentren über die Einstellung des Silica zu Alumina Verhältnisses (SiO₂/Al₂O₃-Verhältnis). Mit abnehmendem Aluminiumgehalt nimmt die Säurestärke des einzelnen Zentrums zu, während gleichzeitig die Anzahl der Zentren abnimmt. Diese beiden gegenläufigen Effekte der Beeinflussung der Azidität überlagern sich gegenseitig. Geeignete Zeolithe sind kommerziell erhältlich oder auf dem Fachmann bekannte Weise herstellbar. Werden in Zeolithen die freien Kationen gegen Protonen ausgetauscht, werden diese als H-Zeolithe bezeichnet. Bevorzugte Zeolithe sind unter der Bezeichnung HY und H-CZB (Hß) erhältlich.

Vorzugsweise werden Zeolithe verwendet, bei denen die Porengröße im Bereich von ≥ 0,56^{∗}0,53 nm bis ≤ 50^{∗}50 nm, bevorzugt im Bereich von ≥ 0,56^{∗}0,56 nm bis ≤ 0,74^{∗}0,74 nm, liegt. Das molare SiO₂/Al₂O₃-Verhältnis wird bei den meisten Zeolithen zwischen 1 und 800 angegeben. In bevorzugten Ausführungsformen weist der Zeolith ein SiO₂/Al₂O₃-Verhältnis im Bereich von ≥ 10 bis ≤ 500, bevorzugt im Bereich von ≥ 30 bis ≤ 200, auf. Das SiO₂/Al₂O₃-Verhältnis im Alumosilikat-Gerüst lässt sich mittels optischer Emissionsspektrometrie mit induktiv gekoppeltem Plasma (ICP-OES) ermitteln. Das SiO₂/Al₂O₃-Verhältnis des Zeolithen HY beträgt beispielsweise 80. Vorzugweise werden Zeolithe verwendet, bei denen die eingestellte Säurestärke im schwachen bis mittleren Bereich liegt. Vorzugsweise werden Zeolithe mit großen Mikroporen oder Mesoporen verwendet. Die Porenweite wird gemäß IUPAC klassifiziert für Mikroporen: 2,0 nm ≥ dp, und für Mesoporen: 2,0 nm < *d*p ≤ 50 nm, wobei *d*p für den Porendurchmesser steht. Dies kann zu einer hohen Selektivität des Katalysators beitragen.

In bevorzugten Ausführungsformen ist ein bifunktioneller Katalysator umfassend Kupfer und ein Alumosilikat (Zeolith) verwendbar. In vorteilhafter Weise zeigte diese Kombination eine gute Selektivität für die Umsetzung zu Dimethoxymethan. Insbesondere ermöglichen diese bifunktionellen Katalysatoren eine Umsetzung unter vergleichsweise milden Reaktionsbedingungen in der Gasphase. Die Beladung des Katalysators mit Kupfer liegt in bevorzugten Ausführungsformen im Bereich von ≥ 0,1 Gew.-% bis ≤ 40 Gew.-%, vorzugsweise im Bereich von ≥ 5 Gew.-% bis ≤ 30 Gew.-%, bevorzugt im Bereich von ≥ 10 Gew.-% bis ≤ 20 Gew.-%, bezogen auf das Alumosilikat oder Metalloxid. Unter Verwendung von Zeolithen umfassend eine Beladung mit 10 Gew.-%, insbesondere 20 Gew.-% Kupfer, bezogen auf das Gewicht des Zeolithen, wurde Dimethoxymethan als Hauptprodukt erhalten. Der Bezug der Beladung auf das Alumosilikat oder Metalloxid bedeutet in anderen Worten, dass zur Herstellung einer Beladung mit 20 Gew.-% Kupfer 0,2 g Kupfer und 1 g Zeolith verwendet werden.

Bifunktionelle Katalysatoren sind in Form von geträgerten Katalysatoren, beispielsweise auf Zeolith geträgertes Kupfer, oder durch physikalisches Mischen der Komponenten herstellbar. Beispielsweise können Metalloxide wie Siliziumdioxid, Aluminiumoxid, Titanoxid, Zeolith und Kombinationen von Siliziumdioxid und Aluminiumoxid als Trägermaterialien durch Trockenimprägnierung oder Nassimprägnierung mit wasserlöslichen Metallsalzen wie Cu(NO₃)₂, AgNO₃ und H₂PtCl₄ bifunktionelle Metalloxidgeträgerte Cu-, Au- und Pt-Katalysatoren hergestellt werden. Auch kann durch Rühren eines Zeoliths in einer wässrigen Cu(NO₃)₂-Lösung ein Ionenaustausch im Zeolith erzielt werden. Nach der Trocken- oder Nassimprägnierung oder dem Ionenaustausch können die Katalysatoren von der flüssigen Phase abgetrennt, gewaschen und getrocknet werden.

Nach der Trocknung bzw. vor der Verwendung zur Umsetzung von Methanol zu Dimethoxymethan wird der Katalysator vorzugsweise kalziniert. In bevorzugten Ausführungsformen des Verfahrens wird der Katalysator bei einer Temperatur im Bereich von ≥ 250°C bis ≤ 1200°C, vorzugsweise bei einer Temperatur im Bereich von ≥ 300°C bis ≤ 600°C, kalziniert. Unter dem Begriff "Kalzinieren" wird vorliegend das Erhitzen mit dem Ziel des Entwässerns, Entgasens und/oder Entfernens von Reststoffen aus der Herstellung verstanden.

Vor der Verwendung zur Umsetzung von Methanol zu Dimethoxymethan wird der Katalysator in bevorzugten Ausführungsformen bei erhöhter Temperatur im Wasserstoffstrom reduziert. Vorzugsweise wird der Katalysator im Wasserstoffstrom bei einer Temperatur im Bereich von ≥ 100°C bis ≤ 900°C, vorzugsweise bei einer Temperatur im Bereich von ≥ 300°C bis ≤ 600°C, reduziert. Hierdurch können Reste von Sauerstoff entfernt werden. Dies ist insbesondere vorteilhaft, da Restsauerstoff in dem System zu einer oxidativen Umsetzung von Methanol zu Dimethoxymethan führen könnte.

Die bifunktionellen Katalysatoren sind insbesondere geeignet, eine Umsetzung des Methanols zu Dimethoxymethan in der Gasphase durchzuführen. Das Verfahren ist kontinuierlich oder diskontinuierlich, drucklos oder unter Druck, in Rohrreaktoren, Strömungsreaktoren oder Wirbelreaktoren durchführbar. Die Umsetzung erfolgt vorzugsweise kontinuierlich in Rohreaktoren wie einem Festbett-Röhrenreaktor oder Wirbelbett-Reaktoren. In Ausführungsformen kann somit ein Verfahren zur nicht-oxidativen Herstellung von Dimethoxymethan und Wasserstoff aus Methanol in Gegenwart eines heterogenen Katalysators zur Verfügung gestellt werden, wobei man kontinuierlich gasförmiges Methanol mit dem bifunktionalem Katalysator in Kontakt bringt. Ein großer Vorteil der Verwendung eines bifunktionalen Katalysators ist, dass die Reaktion in Form einer Ein-Schritt-Synthese erfolgen kann.

Die Reaktion kann beispielsweise in einem Rohrreaktor mit Verdampfer durchgeführt werden. Hierbei kann das gasförmige Methanol insbesondere kontinuierlich geführt mit dem Katalysator in Kontakt gebracht werden. Hierzu kann das Methanol in einem dem Reaktor vorgeschalteten Verdampfer oder Vaporisator, beispielsweise einer Kolonne, verdampft werden. Hierbei kann das Methanol auf die Reaktionstemperatur der Umsetzung, beispielsweise eine Temperatur von 150°C bis 200°C, gebracht werden. Anschließend kann das Methanol mit einem Trägergas vermischt werden. Als Trägergas sind inerte Gase wie Argon, vorzugsweise Stickstoff bevorzugt. Eine möglichst homogene Vermischung von Methanol und Trägergas ist dabei bevorzugt.

Das Methanol kann als Reingas oder verdünnt mit einen Trägergas über den Katalysator geleitet werden. In bevorzugten Ausführungsformen des Verfahrens bringt man das Methanol als Reingas oder mit einem Trägergas verdünnt mit dem Katalysator in Kontakt, wobei der Anteil des Methanols im Gasstrom vorzugsweise im Bereich von ≥ 0,1 Vol.-% bis ≤ 100 Vol.-%, liegt. Bei Verwendung eines Trägergases liegt der Anteil des Methanols im Gasstrom vorzugsweise im Bereich von ≥ 0,1 Vol.-% bis ≤ 80 Vol.-%, bevorzugt im Bereich von ≥ 10 Vol.-% bis ≤ 60 Vol.-%, bezogen auf das Gesamtvolumen des Gasstroms. Es kann bevorzugt sein, reines Methanol zu verwenden.

In bevorzugten Ausführungsformen bringt man das Methanol mit einer Raumgeschwindigkeit (GHSV, Gas Hourly Space Velocity) im Bereich von ≥ 1.000 mL/h^{∗}g bis ≤ 200.000 mL/h^{∗}g, vorzugsweise im Bereich von ≥ 2.500 mL/h^{∗}g bis ≤ 50.000 mL/h^{∗}g, vorzugsweise im Bereich von ≥ 5.000 mL/h^{∗}g bis ≤ 15.000 mL/h^{∗}g, mit dem Katalysator in Kontakt. Insbesondere in diesen Bereichen konnten eine hohe Selektivität verbunden mit erhöhten Ausbeuten an Dimethoxymethan erhalten werden.

In bevorzugten Ausführungsformen bringt man das Methanol bei einer Temperatur im Bereich von ≥ 100°C bis ≤ 900°C, vorzugsweise bei einer Temperatur im Bereich von ≥ 140°C bis ≤ 500°C, bevorzugt bei einer Temperatur im Bereich von ≥ 140°C bis ≤ 300°C, insbesondere bei einer Temperatur im Bereich von ≥ 140°C bis ≤ 200°C oder von ≥ 150°C bis ≤ 200°C, mit dem Katalysator in Kontakt. In vorteilhafter Weise kann die Umsetzung bei niedriger Temperatur in der Gasphase durchgeführt werden. Durch geringere Temperaturen sind geringere Energiekosten erforderlich, wodurch der Herstellungsprozess technisch einfacher und wirtschaftlich attraktiver wird.

In bevorzugten Ausführungsformen bringt man das Methanol mit einem Druck im Bereich von ≥ 0,1 MPa bis ≤ 0,8 MPa, bevorzugt mit einem Druck im Bereich von ≥ 0,1 MPa bis ≤ 0,3 MPa, mit dem Katalysator in Kontakt. Drücke im Bereich des Atmosphärendrucks bis leicht erhöhten Drücken erbrachten gute Ergebnisse.

Die aus dem Verfahren zur nicht-oxidativen Herstellung von Dimethoxymethan resultierenden Reaktionsgemische können nach herkömmlichen Methoden aufgereinigt werden, beispielsweise thermisch mittels destillativer oder rektifikativer Verfahren. Durch das erfindungsgemäße Verfahren ist Dimethoxymethan durch nicht-oxidative Umsetzung von Methanol auf wirtschaftliche Weise herstellbar. Dimethoxymethan ist industriell vielfach verwendbar und lässt sich insbesondere zu höheren kurzkettigen Oxymethylenethern umsetzen, welche als Kraftstoff-Zusatz dienen können.

Insgesamt kann ein Verfahren zur Verfügung gestellt werden, das unter Verwendung eines gut verfügbaren Eduktes eine Herstellung von Dimethoxymethan erlaubt. Die Umsetzung konnte kontinuierlich in der Gasphase bei niedriger Temperatur mit hoher Selektivität geführt werden.

Wenn nicht anders ausgeführt, weisen die verwendeten technischen und wissenschaftlichen Ausdrücke die Bedeutung auf, wie sie gemeinhin von einem Durchschnittsfachmann in dem Gebiet, zu dem diese Erfindung gehört, verstanden wird.

Beispiele, die der Veranschaulichung der vorliegenden Erfindung dienen, sind nachstehend angegeben.

### Gaschromatographie:

Die Reaktionsgemische wurden mittels Online-Gaschromatographie mithilfe eines Bruker scion 456-GC und einer HP-PLOT U (30 m Länge, 0,32 mm Durchmesser) (Agilent) analysiert und quantifiziert.

### Beispiel 1

### Katalysator-Herstellung mittels Trockenimprägnierung

Siliciumdioxid (Saint Gobain) Aluminiumoxid (Saint Gobain), Titanoxid (Saint Gobain), Siliziumdioxid dotiert mit ZrO₂ und TiO₂ (Saint Gobain), Zeolith (Clariant) / HY (Alfa Aesar), Zirkoniumdioxid (Alfa Aesar) und SIRALOX (5% Siliciumdioxid und 95% Aluminiumoxid, Sasol Germany GmBH) wurden vor dem Imprägnierungsprozess bei 550 °C für 4 Stunden kalziniert, um Wasser aus den Poren zu entfernen. Wasserlösliche Metallsalze wie Cu(NO₃)₂ (ACROS ORGANICS), HAuCl₄ (Sigma Aldrich), AgNO₃ (Sigma Aldrich) und H₂PtCl₄ (Sigma Aldrich) wurden als Vorstufen zur Herstellung von Metalloxidgeträgerten Cu-, Au-, Ag- und Pt-Katalysatoren verwendet. Zunächst wurden die berechneten Mengen als Vorläufer (bezogen auf die Metallbeladung) in Wasser gelöst, bis sich eine transparente Lösung ergab. Die zuvor gemessene Wasseradsorptionskapazität verschiedener Trägermaterialien wurde dazu genutzt, die zugegebene Wassermenge zu berechnen. Danach wurde der transparenten Lösung unter intensivem Rühren der Träger zugegeben. Die resultierenden Proben wurden über Nacht stehengelassen und bei Raumtemperatur unter atmosphärischen Bedingungen langsam getrocknet. Eine finale Trocknung wurde in einem Ofen für 12 Stunden bei 120°C vorgenommen. Schließlich wurde die getrocknete Probe 4 Stunden bei 450 °C kalziniert.

### Beispiel 2

### Herstellung von geträgerten Kupfer-Katalysatoren mit Phosphordotierung

Die Herstellung von Cu auf TiO₂ umfasste die folgenden Schritte, wobei die Synthese sowohl für Cu auf TiO₂ als auch für andere Katalysatoren bei denen Cu verwendet wurde, in dieser Weise durchgeführt wurde. In einem ersten Schritt wurde Cu auf TiO₂ imprägniert. Hierzu wurden 0,377 g Cu (NO₃)₂ · 3 H₂O in 710 µl Wasser gelöst und 0,89 g TiO₂ wurden zugegeben und stark gerührt. Die Probe wurde über Nacht zum Trocknen an der Luft gelassen und dann bei 120 °C für 12 Stunden im Ofen getrocknet. Die resultierende Probe wurde mit wässriger Phosphorsäure, 1,8 ml 85%-ige H₃PO₄ verdünnt mit 690 µl Wasser, zum Zwecke der zweiten Imprägnierung behandelt und über Nacht an der Luft getrocknet. Anschließend fand ein weiteres 12-stündiges Trocknen im Ofen bei 120 °C statt. Schlussendlich wurde der Katalysator bei 450 °C für 3 h in einem Muffelofen mit einer Heizrate von 5 °C / min kalziniert.

### Beispiel 3

### Herstellung von Kupfer geträgert auf Zeolithen mittels Ionenaustausch

Der Zeolith HCZB150 (Hβ150) (Clariant) wurde für 4 Stunden in 20 ml/g einer wässrigen 0,5 M Cu(NO₃)₂-Lösung bei 80 °C gerührt. Das Material wurde gefiltert und mehrere Male mit entsalztem Wasser gewaschen und danach bei 120°C getrocknet. Das Verfahren wurde dreimal wiederholt und der erhaltene Katalysator schließlich bei 450 °C für 4 Stunden unter statischer Luft kalziniert.

### Beispiele 4-13

### Nicht-oxidative Herstellung von Dimethoxymethan aus Methanol

Die katalytischen Tests wurden in einem kontinuierlichen Gasphasenaufbau durchgeführt. Das Katalysatorbett umfasste eine Mischung von Katalysator und Siliciumcarbid, die eine homogene Wärmeverteilung während der gesamten Reaktion ermöglicht und eine lokale Überhitzung verhindert.

Wenn nicht abweichend angegeben betrug das Massenverhältnis von Katalysator zu Siliciumcarbid 1:9 und die Gesamtmasse betrug 1 g. Ein Festbettreaktor (Innendurchmesser 6 mm), der in einem Röhrenofen angeordnet war, wurde für die Umsetzung verwendet. Die Reaktionstemperatur wurde durch ein Thermoelement innerhalb des Reaktors in einem Bereich von 120 bis 300 °C geregelt. Der Katalysator wurde zwischen zwei Schichten Quarzwolle innerhalb des Rohrreaktors angeordnet, und die Höhe des Katalysators wurde gemessen, um die Kontaktzeit zu bestimmen. Der Katalysator wurde, wenn nicht abweichend angegeben, bei 450°C in dem Reaktor in einem reinen H₂-Strom von 20 ml/min vor der Reaktion vorreduziert.

Flüssiges Methanol wurde mit einer Rate von 0,008 ml/min in die Rohrleitung gepumpt und in einer mit Heizband geheizten Kapillare verdampft, bevor es in den Reaktor eintrat. Das gasförmige Methanol wurde mit einem N₂-Strom von 20 ml/min verdünnt. Der molare Anteil von Methanol in dem Zufuhrstrom betrug, wenn nicht abweichend angegeben, etwa 20% und die Raumgeschwindigkeit 14.909 ml/(h^{∗}g). Die Raumgeschwindigkeit wurde aus der Gesamtgasflussrate dividiert durch die Katalysatormasse ermittelt. Der Produktgasstrom, der Produkte und nicht umgesetztes Methanol enthielt, wurde wie oben beschrieben online gaschromatographisch bestimmt.

Die Bedingungen, Umsatz und Selektivität der einzelnen Umsetzungen mit verschiedenen Katalysatoren sind in der folgenden Tabelle 1 zusammengefasst. Der nominale Metallgehalt ist in Gewichtsprozent angegeben. Umwandlung und Selektivität werden auf der Basis von Kohlenstoffmolekülen berechnet.

**Tabelle 1: Selektivität der nicht-oxidativen Herstellung von Dimethoxymethan**

| Beispiel | Reaktionsbedingungen | | | | Selektivität [%] |
|---|---|---|---|---|---|
| | Katalysator | Temperatur [°C] | GHSV [mL/h*g ] | Umsatz [%] | |
| 4 | 0,3% Pt/Al₂O₃ | 240 | 14909 | 7,1 | 6,2 |
| 5 | MoS₂ | 280 | 14909 | 2,9 | 5,7 |
| 6 nicht erfindungsgemäß | 20% Cu-1,5% P/SiO₂ | 240 | 14909 | 3,9 | 21,7 |
| 7 | 20% Cu/Hβ | 160 | 14909 | 6,5 | 51,8 |
| 8 | 20% Cu/Hβ | 140 | 4970 | 4,8 | 49,6 |
| 9 | 20% Cu/HY | 160 | 14909 | 2,3 | 51,6 |
| 10 | 20% Cu/HY | 180 | 5584 | 5,6 | 56,2 |
| 11 | 10%Cu/ SIRALOX | 200 | 14909 | 37,7 | 16,0 |
| 12 | 10%Cu-1%P/TiO₂ | 280 | 14909 | 3,1 | 7,4 |
| 13 | 0,8%Ag/ZrO₂ | 240 | 14909 | 5,2 | 4,9 |

Wie man der Tabelle 1 entnimmt, wurde bei Temperaturen von 140-180 °C insbesondere unter Verwendung von bifunktionalen mit Kupfer beladenen Zeolith-Katalysatoren eine sehr gute Selektivität für Dimethoxymethan festgestellt.

### Vergleichsbeispiele 14-16 und Blindproben 17-19

Die nicht-oxidative Gasphasen-Herstellung von Dimethoxymethan aus Methanol wurde wie für die Beispiele 4-13 beschrieben mit einem bifunktionalen mit 0,8 Gew.-% Gold beladenen Zirkuniumoxid/Siliciumoxid-Katalysator unter Vwendung von 8,3 mol% Methanol wiederholt. Weiterhin wurden Blindproben ohne Katalysator lediglich mit Siliciumcarbid durchgeführt, um den Einfluss des Katalysatorbetts zu bestimmen.

In der folgenden Tabelle 2 sind die Bedingungen und Selektivitäten der Gegenbeispiele und Blindproben zusammengefasst.

**Tabelle 2: Gegenbeispiele 14-16 und Blindproben 17-19**

| Beispiel | Reaktionsbedingungen | | | | Selektivität [%] |
|---|---|---|---|---|---|
| | Katalysator | Temperatur [°C] | GHSV [mL/h*g] | Umsatz [%] | |
| 14 | 0,8% Au-ZrO₂/ SiO₂ | 180 | 4184 | 1,2 | 1,1 |
| 15 | 0,8% Au-ZrO₂/ SiO₂ | 200 | 4184 | 2,2 | 1,5 |
| 16 | 0,8% Au-ZrO₂/ SiO₂ | 240 | 4184 | 11,4 | 0,7 |
| 17 | - | 160 | 14909 | 0 | 0 |
| 18 | - | 240 | 14909 | 0 | 0 |
| 19 | - | 320 | 14909 | 0,2 | 0 |

Wie man der Tabelle 2 entnimmt, wurde unter Verwendung von Gold keine ausreichende Dehydrierung des Methanols und Umsetzung zu Dimethoxymethan beobachtet. Siliciumcarbid beeinflusste die Umsetzung nicht. Bei 320 °C wurde eine Zersetzung des Methanols und weiter eine Entstehung von Methylformiat, Dimethylether, Methan und Kohlendioxid beobachtet.

Die Ergebnisse zeigen insgesamt, dass eine nicht-oxidativen Herstellung von Dimethoxymethan aus Methanol in Gegenwart eines heterogenen Katalysators in der Gasphase mit guter Selektivität zur Verfügung gestellt werden kann.

Die dieser Patentanmeldung zu Grunde liegende Erfindung entstand in einem Projekt, welches unter dem Förderkennzeichen 162991553100014 (Kopernikus P2X) vom BMBF gefördert wurde.

## Patentansprüche

1. Verfahren zur nicht-oxidativen Herstellung von Dimethoxymethan aus Methanol in Gegenwart eines heterogenen Katalysators, wobei man gasförmiges Methanol mit dem Katalysator in Kontakt bringt, **dadurch gekennzeichnet, dass** der Katalysator ein bifunktioneller Katalysator ist, umfassend:
a) Molybdänsulfid, oder
b)
- ein Metall oder eine Metallverbindung ausgewählt aus der Gruppe umfassend Kupfer, Silber, Platin und/oder Molybdänsulfid, und
- ein Alumosilikat, vorzugsweise ein Zeolith, oder ein Metalloxid ausgewählt aus der Gruppe umfassend Titanoxid, Zirkonoxid und/oder Aluminiumoxid.

2. Verfahren nach Anspruch 1, **dadurch gekennzeichnet, dass** das Alumosilikat und das Metalloxid optional mit Phospohor oder Schwefel dotiert sind.

3. Verfahren nach Anspruch 1 oder 2, **dadurch gekennzeichnet, dass** der Katalysator ein bifunktioneller Katalysator umfassend Kupfer und ein Alumosilikat ist.

4. Verfahren nach einem der vorhergehenden Ansprüche, **dadurch gekennzeichnet, dass** die Beladung des Katalysators mit Kupfer im Bereich von ≥ 0,1 Gew.-% bis ≤ 40 Gew.-%, vorzugsweise im Bereich von ≥ 5 Gew.-% bis ≤ 30 Gew.-%, bevorzugt im Bereich von ≥ 10 Gew.-% bis ≤ 20 Gew.-%, bezogen auf das Alumosilikat oder Metalloxid, liegt.

5. Verfahren nach einem der vorhergehenden Ansprüche, **dadurch gekennzeichnet, dass** der Katalysator im Wasserstoffstrom bei einer Temperatur im Bereich von ≥ 100°C bis ≤ 900°C, vorzugsweise bei einer Temperatur im Bereich von ≥ 300°C bis ≤ 600°C, reduziert wird.

6. Verfahren nach einem der vorhergehenden Ansprüche, **dadurch gekennzeichnet, dass** der Katalysator bei einer Temperatur im Bereich von ≥ 250°C bis ≤ 1200°C, vorzugsweise bei einer Temperatur im Bereich von ≥ 300°C bis ≤ 600°C, kalziniert wird.

7. Verfahren nach einem der vorhergehenden Ansprüche, **dadurch gekennzeichnet, dass** man das Methanol als Reingas oder mit einem Trägergas verdünnt mit dem Katalysator in Kontakt bringt, wobei der Anteil des Methanols im Gasstrom vorzugsweise im Bereich von ≥ 0,1 Vol.-% bis ≤ 100 Vol.-%, bevorzugt im Bereich von ≥ 10 Vol.-% bis ≤ 60 Vol.-%, liegt.

8. Verfahren nach einem der vorhergehenden Ansprüche, **dadurch gekennzeichnet, dass** man das Methanol mit einer Raumgeschwindigkeit (GHSV, Gas Hourly Space Velocity) im Bereich von ≥ 1.000 mL/h^{∗}g bis ≤ 200.000 mL/h^{∗}g, vorzugsweise im Bereich von ≥ 2.500 mL/h^{∗}g bis ≤ 50.000 mL/h^{∗}g, bevorzugt im Bereich von ≥ 5.000 mL/h^{∗}g bis ≤ 15.000 mL/h^{∗}g, mit dem Katalysator in Kontakt bringt.

9. Verfahren nach einem der vorhergehenden Ansprüche, **dadurch gekennzeichnet, dass** man das Methanol bei einer Temperatur im Bereich von ≥ 100°C bis ≤ 900°C, vorzugsweise bei einer Temperatur im Bereich von ≥ 140°C bis ≤ 500°C, bevorzugt bei einer Temperatur im Bereich von ≥ 140°C bis ≤ 200°C, mit dem Katalysator in Kontakt bringt.

10. Verfahren nach einem der vorhergehenden Ansprüche, **dadurch gekennzeichnet, dass** man das Methanol mit einem Druck im Bereich von ≥ 0,1 MPa bis ≤ 0,8 MPa, bevorzugt mit einem Druck im Bereich von ≥ 0,1 MPa bis ≤ 0,3 MPa, mit dem Katalysator in Kontakt bringt.

## Claims

1. Process for the non-oxidative preparation of dimethoxymethane from methanol in the presence of a heterogeneous catalyst, by contacting gaseous methanol with the catalyst, **characterized in that** the catalyst is a bifunctional catalyst comprising:
a) molybdenum sulfide, or
b)
- a metal or a metal compound selected from the group encompassing copper, silver, platinum and/or molybdenum sulfide, and
- an aluminosilicate, preferably a zeolite, or a metal oxide selected from the group encompassing titanium oxide, zirconium oxide and/or aluminium oxide.

2. Process according to Claim 1, **characterized in that** the aluminosilicate and the metal oxide optionally have doping with phosphorus or sulfur.

3. Process according to Claim 1 or 2, **characterized in that** the catalyst is a bifunctional catalyst comprising copper and an aluminosilicate.

4. Process according to any one of the preceding claims, **characterized in that** the loading of the catalyst with copper is in the range from ≥ 0.1 wt.% to ≤ 40 wt.%, preferably in the range from ≥ 5 wt.% to ≤ 30 wt.%, more preferably in the range from ≥ 10 wt.% to ≤ 20 wt.%, based on the aluminosilicate or metal oxide.

5. Process according to any one of the preceding claims, **characterized in that** the catalyst is reduced in a stream of hydrogen at a temperature in the range from ≥ 100°C to ≤ 900°C, preferably at a temperature in the range from ≥ 300°C to ≤ 600°C.

6. Process according to any one of the preceding claims, **characterized in that** the catalyst is calcined at a temperature in the range from ≥ 250°C to ≤ 1200°C, preferably at a temperature in the range from ≥ 300°C to ≤ 600°C.

7. Process according to any one of the preceding claims, **characterized in that** the methanol is contacted as pure gas or diluted with a carrier gas with the catalyst, the fraction of the methanol in the gas stream being preferably in the range from ≥ 0.1 vol.% to ≤ 100 vol.%, more preferably in the range from ≥ 10 vol.% to ≤ 60 vol.%.

8. Process according to any one of the preceding claims, **characterized in that** the methanol is contacted at a space velocity (GHSV, gas hourly space velocity) in the range from ≥ 1000 mL/h*g to ≤ 200 000 mL/h*g, preferably in the range from ≥ 2500 mL/h*g to ≤ 50 000 mL/h*g, more preferably in the range from ≥ 5000 mL/h*g to ≤ 15 000 mL/h*g, with the catalyst.

9. Process according to any one of the preceding claims, **characterized in that** the methanol is contacted at a temperature in the range from ≥ 100°C to ≤ 900°C, preferably at a temperature in the range from ≥ 140°C to ≤ 500°C, more preferably at a temperature in the range from ≥ 140°C to ≤ 200°C, with the catalyst.

10. Process according to any one of the preceding claims, **characterized in that** the methanol is contacted at a pressure in the range from ≥ 0.1 MPa to ≤ 0.8 MPa, preferably at a pressure in the range from ≥ 0.1 MPa to ≤ 0.3 MPa, with the catalyst.

## Revendications

1. Procédé de fabrication non oxydative de diméthoxyméthane à partir de méthanol en présence d'un catalyseur hétérogène, dans lequel on met du méthanol gazeux en contact avec le catalyseur, **caractérisé en ce que** le catalyseur est un catalyseur bifonctionnel comprenant :
a) du sulfure de molybdène, ou
b)
- un métal ou un composé métallique choisi dans le groupe comprenant le cuivre, l'argent, le platine et/ou le sulfure de molybdène, et
- un aluminosilicate, de préférence une zéolithe, ou un oxyde métallique choisi dans le groupe consistant en l'oxyde de titane, l'oxyde de zirconium et/ou l'oxyde d'aluminium.

2. Procédé selon la revendication 1, **caractérisé en ce que** l'aluminosilicate et l'oxyde métallique sont éventuellement dopés par du phosphore ou du soufre.

3. Procédé selon la revendication 1 ou 2, **caractérisé en ce que** le catalyseur est un catalyseur bifonctionnel comprenant du cuivre et un aluminosilicate.

4. Procédé selon l'une des revendications précédentes, **caractérisé en ce que** la charge du catalyseur en cuivre est comprise dans la plage de ≥ 0,1 % en poids à ≤ 40 % en poids, de préférence dans la plage de ≥ 5 % en poids à ≤ 30 % en poids, préférentiellement dans la plage de ≥ 10 % en poids à ≤ 20 % en poids, par rapport à l'aluminosilicate ou à l'oxyde métallique.

5. Procédé selon l'une des revendications précédentes, **caractérisé en ce que** le catalyseur est réduit dans le courant d'hydrogène à une température dans la plage de ≥ 100 °C à ≤ 900 °C, de préférence à une température dans la plage de ≥ 300 °C à ≤ 600 °C.

6. Procédé selon l'une des revendications précédentes, **caractérisé en ce que** le catalyseur est calciné à une température dans la plage de ≥ 250 °C à ≤ 1 200 °C, de préférence à une température dans la plage de ≥ 300 °C à ≤ 600 °C.

7. Procédé selon l'une des revendications précédentes, **caractérisé en ce qu'**on met en contact le méthanol, sous forme gaz pur ou dilué avec un gaz support, avec le catalyseur, la proportion du méthanol dans le courant gazeux étant de préférence de ≥ 0,1 % en volume à ≤ 100 % en volume, de préférence dans la plage de ≥ 10 % en volume à ≤ 60 % en volume.

8. Procédé selon l'une des revendications précédentes, **caractérisé en ce qu'**on met en contact le méthanol avec le catalyseur à une vitesse spatiale horaire (GHSV, Gas Hourly Space Velocity) dans la plage de ≥ 1 000 ml/h*g à ≤ 200 000 ml/h*g, de préférence dans la plage de ≥ 2 500 ml/h*g à ≤ 50 000 ml/h*g, préférentiellement dans la plage de ≥ 5 000 ml/h*g à ≤ 15 000 ml/h*g.

9. Procédé selon l'une des revendications précédentes, **caractérisé en ce qu'**on met le méthanol en contact avec le catalyseur à une température dans la plage de ≥ 100 °C à ≤ 900 °C, de préférence à une température dans la plage de ≥ 140 °C à ≤ 500 °C, préférentiellement à une température dans la plage de ≥ 140 °C à ≤ 200 °C.

10. Procédé selon l'une des revendications précédentes, **caractérisé en ce qu'**on met le méthanol en contact avec le catalyseur sous une pression dans la plage de ≥ 0,1 MPa à ≤ 0,8 MPa, préférentiellement sous une pression dans la plage de ≥ 0,1 MPa à ≤ 0,3 MPa.
